(19) Europäisches Patentamt / European Patent Office / Office européen des brevets

(11) **EP 3 282 007 B1**

(12) **EUROPEAN PATENT SPECIFICATION**

(45) Date of publication and mention
of the grant of the patent:
**23.09.2020 Bulletin 2020/39**

(51) Int Cl.:
**C12N 1/14** (2006.01)  **A01N 63/34** (2020.01)
**A01P 5/00** (2006.01)

(21) Application number: **16776829.0**

(86) International application number:
**PCT/KR2016/003551**

(22) Date of filing: **06.04.2016**

(87) International publication number:
**WO 2016/163726 (13.10.2016 Gazette 2016/41)**

(54) **ASPERGILLUS NIGER**

ASPERGILLUS NIGER

SOUCHE D'ASPERGILLUS NIGER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.04.2015 KR 20150048571**

(43) Date of publication of application:
**14.02.2018 Bulletin 2018/07**

(73) Proprietors:
• **Industry Foundation of Chonnam National
University
Gwangju 61186 (KR)**
• **Korea Research Institute of Chemical
Technology
Daejeon 34114 (KR)**
• **Korea Research Institute of Bioscience and
Biotechnology
Daejeon 34141 (KR)**

(72) Inventors:
• **KIM, Jin Cheol
Daejeon 34119 (KR)**
• **KIM, Hun
Jinju-si
Gyeongsangnam-do 52650 (KR)**
• **SHIN, Kee-Sun
Daejeon 34141 (KR)**
• **JANG, Ja Yeong
Yeonggwang-gun
Jeollanam-do 57068 (KR)**
• **CHOI, Gyung Ja
Daejeon 34049 (KR)**
• **CHOI, Yong Ho
Daejeon 34119 (KR)**
• **JANG, Kyoung Soo
Daejeon 34164 (KR)**
• **KIM, Mi Bang
Daejeon 34141 (KR)**

(74) Representative: **Dehns
St. Bride's House
10 Salisbury Square
London EC4Y 8JD (GB)**

(56) References cited:
CN-A- 1 756 482      CN-A- 101 182 469
JP-A- 2005 082 530      KR-A- 20060 002 789

• ONONUJU- C. C. ET AL.: 'Efficacy of Using
Fungus (Aspergillus Niger) as an Antagonist and
Synthetic Menaticide (Furadan) in the
Management of Meloidogyne Incognita in Okra'
JOURNAL OF ADVANCES IN AGRICULTURAL)
SCIENCE AND TECHNOLOGY vol. 2, no. 6, 2014,
pages 86 - 88, XP055319965

**Description**

[Technical Field]

The present invention relates to an *Aspergillus niger* F22 strain having a nematicidal activity against plant-parasitic nematodes and a use thereof, and more particularly, to an *Aspergillus niger* F22 strain having a nematicidal activity against plant-parasitic nematodes, a nematicidal microorganism agent against plant-parasitic nematodes including the strain, or a spore, a fungal hyphal mass or a culture broth thereof as an active ingredient, a method for controlling plant-parasitic nematodes, which includes a step of treating a crop, a crop seed, or a field with the nematicidal microorganism agent, and a method for the preparation of a nematicidal microorganism agent against plant-parasitic nematodes, which includes culturing the strain.

[Background Art]

Typical nematodes causing damages to main garden crops, fruits, and vegetables in horticultural facilities in Korea are known as plant-parasitic nematodes including root-knot nematodes (i.e., *Meloidogyne* spp.). In particular, root-knot nematodes in the greenhouse soil cause serious damage to farm crops. The plant-parasitic nematode includes *Meloidogyne* spp., *Globodera* spp., *Anguina* spp., *Ditylenchus* spp., *Aphalenchoides* spp., *Pratylenchus* spp., *Tylenchorhychus* spp., *Rotylenchulus* spp., etc., depending on the damage patterns and hosts. *Meloidogyne incognita, Meloidogyne hapla, Meloidogyne javanica,* and *Meloidogyne arenaria* in root-knot nematodes are major nematodes adversely affecting agriculture.

In recent years, in order to control plant parasitic nematodes, cultural, physical, or chemical control has been used. The cultural control is realized using fresh water, fallow ground, dried soil, deep plowing, transported soil, rice rotation cultivation, resistant cultivars, and the like, the physical control is realized through heat treatment (steam, dry heat, and hot water soaking methods), sun heat sterilization, and the like, and the chemical control is realized by treatment of chemical agents such as carbofuran, fosthiazate, and the like to control nematodes. The cultural control is expensive or limited in application depending on the regional conditions and crops, and the resistant cultivars have a drawback in that their applications are particularly limited to crops, resulting in low practicality. Although the chemical nematicides has a high control effect against plant parasitic nematodes, they mainly organophosphorous or carbamates, are highly toxic when they remain in soil, cause cumulative damage to the crops as well as environmental pollution due to bactericidal actions against effective microorganisms in the soil, which are beneficial for the growth of crops, and gradually make soil infertile. Therefore, the use of chemical pesticides has been avoided due to the problems such as environmental pollution, destruction of the ecosystem, the pesticide poisoning to humans and animals, and an increase in demand for environmentally friendly agricultural products. Also, Methyl bromide which has been generally used as a soil fumigant to control nematodes will be banned due to depletion of the ozone layer(Caboni et al. (2013) J. Agric. Food Chem. 61, 1794-1803). Therefore, there is a demand for development of nematicides which are safer for the environments and may be replaced with the aforementioned agricultural pesticides.

In recent years, among the methods for controlling plant-parasitic nematodes in an environmentally friendly manner, there is a method using microorganisms. Some studies showed that, as microorganisms grow, they produce various secondary metabolites and directly act on plant pathogens to exhibit various antibacterial and insecticidal activities (Zhao et al. (2014) Antagonistic action of Bacillus subtillis strain SG6 on Fusarium graminearum. PLoS one. 9(3), e92486). Therefore, research is under way to isolate strains having a nematicidal activity through screening for nematicidal activities of various culture broths of microorganisms and develop strains for controlling diseases caused by plant-parasitic nematodes using the same.

Meanwhile, Korean Unexamined Patent Publication No. 2006-0002789 discloses a 'nematicide,' and Korean Unexamined Patent Publication No. 2010-0116562 discloses a *'Bacillus velezensis* G341 strain and method for controlling plant diseases using the same.' However, an *Aspergillus niger* F22 strain having a nematicidal activity against plant-parasitic nematodes and a use thereof are not found, as disclosed in the present invention. CN 101 182 469 discloses strain ANUV90 which has a nematicidal activity against *Meloidogyne* spp.. JP 2005 082530 discloses strains JAM4055 and JAM4054 which have a nematicidal activity against *Bursaphelenchus xylophilus.*

[Disclosure]

[Technical Problem]

Therefore, the present invention is designed to solve the problems of the prior art, and the present inventors have found that, among 61 fungi, an *Aspergillus niger* F22 (KCTC 12771BP) is a strain that has a nematicidal activity against plant-parasitic nematode larvae under laboratory conditions and remarkably suppresses the onset of root-knot nematode diseases even under greenhouse conditions using tomato crops. Also, the present inventors have confirmed that, when 5 basic formulations such as suspension concentrate (SC), suspension microbial (SM), absorbent granule (absorbent GR), powdery granule (powdery GR), and wettable powder (WP) formulations are prepared to check a control activity against *Meloidogyne incognita,* the wettable powder formulation has the most excellent control value. Therefore, the present inventors have confirmed that the strain of the present invention may replace the chemical pesticides as a biological pesticide for controlling plant-parasitic nematodes, and may be provided as a groundbreaking biological control agent which may solve the problems caused by the environmental pollution. Therefore, the present invention has been completed based on these facts.

[Technical Solution]

To solve the above problems, according to an aspect of the present invention, there is provided an *Aspergillus niger* F22 strain having a nematicidal activity against plant-parasitic nematodes.

According to another aspect of the present invention, there is provided a nematicidal microorganism agent activity against plant-parasitic nematodes, which includes the strain, or a spore, a fungal hyphal mass or a culture broth thereof as an active ingredient.

According to still another aspect of the present invention, there is provided a method for controlling plant-parasitic nematodes, which includes a step of treating a crop, a crop seed or a field with the microorganism agent.

According to yet another aspect of the present invention, there is provided a method for preparation of a nematicidal microorganism agent against plant-parasitic nematodes, which includes culturing the strain.

[Advantageous Effects]

The *Aspergillus niger* F22 strain of the present invention has a nematicidal activity against *Meloidogyne incognita* in a concentration-dependent manner in an experiment in the greenhouse as well as an *in vitro* experiment, and thus is considered to have a high potential for development as a control agent against the plant-parasitic nematodes. Even when the strain of the present invention is prepared into a wettable powder formulation and *Meloidogyne incognita* is treated with the wettable powder formulation, the strain of the present invention has an excellent control effect. Therefore, when subsequent optimal fermentation and formulation optimization processes are carried out, the strain of the present invention can be used a biological pesticide, which is a novel microbial nematicide, instead of the chemical pesticides. Accordingly, the strain of the present invention can be very effectively used as a groundbreaking biological control agent that can solve the problems caused by the environmental pollution.

[Brief Description of The Drawings]

FIG. 1 is a graph illustrating nematicidal activities of selected strains against *Meloidogyne incognita* (*M. incognita*) when *M. incognita* is treated with various culture filtrate concentrations of the strain according to the present invention.

FIG. 2 shows results of comparing a shape of dead *M. incognita,* which is treated with each of 10% culture filtrates of the selected strains, with a shape of an untreated group according to the present invention.

FIG. 3 shows a phylogenetic analysis using (A) ITS regions and (B) 26S RNA sequences of the selected strain according to the present invention.

FIG. 4 is a graph illustrating nematicidal activities of the *Aspergillus niger* F22 strain depending on a culture medium according to the present invention.

FIG. 5 is a graph illustrating disease control activities of the *Aspergillus niger* F22 strain against root-knot nematode disease caused by *M. incognita* when tomato plants were treated with a culture filtrate of the *Aspergillus niger* F22 strain prior to infection of *M. incognita* according to the present invention.

FIG. 6 shows results of comparing shapes of roots of tomato plants, which are treated with a 10-fold dilute solution of the culture filtrate of the *Aspergillus niger* F22 strain, with a shape of the untreated group according to the present invention.

FIG. 7 is a graph illustrating control values of basic formulations, which are prepared using the culture broth of the *Aspergillus niger* F22 strain, against *Meloidogyne incognita* according to the present invention.

FIG. 8 shows results of comparing shapes of roots of tomato plants in groups, in which *M. incognita* is treated with 100-fold and 50-fold dilute solutions of a wettable powder agent of the *Aspergillus niger* F22 strain, with shapes of roots of tomato plants in an untreated group and groups in which *M. incognita* is treated with a 3,000-fold dilute solution of abamectin (All Star) according to the present invention.

FIG. 9 is a graph illustrating nematicidal activities of the culture filtrates of the *Aspergillus niger* F22 strain against *Meloidogyne hapla* and *Bursaphelenchus xylophilus* depending on concentration.

[Detailed Description of Preferred Embodiments]

To achieve the objectives, the present invention provides an *Aspergillus niger* F22 strain having a nematicidal activity against plant-parasitic nematodes.

In the present invention, it is confirmed that, among 61 fungi, a culture filtrate of a certain strain exhibits a nematicidal activity against plant-parasitic nematode larvae under laboratory conditions, and also remarkably suppresses the onset of root-knot nematode diseases even under greenhouse conditions using tomato crops. In this case, the strain was identified as an *Aspergillus niger* F22 strain. The *Aspergillus niger* F22 strain was deposited in the Korea Research Institute of Bioscience and Biotechnology on March 18, 2015 (Accession Number: KCTC 12771BP)

In the strain according to one exemplary embodiment of the present invention, the plant-parasitic nematodes may include *Bursaphelenchus xylophilus* or *Meloidogyne* spp. Here, the preferred *Meloidogyne* spp. may include one or more selected from *Meloidogyne incognita, Meloidogyne hapla, Meloidogyne javanica, Meloidogyne arenaria,* and *Meloidogyne incognita* (*M. incognita*), but the present invention is not limited thereto.

In the strain according to one exemplary embodiment of the present invention, the most preferred plant-parasitic nematodes may include *Meloidogyne incognita, Meloidogyne hapla* or *Bursaphelenchus xylophilus,* but the present invention is not limited thereto.

Also, the present invention provides a nematicidal microorganism agent against plant-parasitic nematodes, which includes the strain, or a spore, a fungal hyphal mass or a culture broth thereof as an active ingredient.

The microorganism agent may include the *Aspergillus niger* F22 strain having a nematicidal activity against plant-parasitic nematodes, or a spore, a fungal hyphal mass or a culture broth thereof as an active ingredient.

Preferably, the microorganism agent may be a suspension concentrate (SC), suspension microbial (SM), absorbent granule (absorbent GR), powdery granule (powdery GR) or wettable powder (WP) formulations, most preferably a wettable powder formulation, but the present invention is not limited thereto.

The microorganism agent includes a culture broth prepared by culturing each of the aforementioned strains under the aforementioned culture conditions, and thus may be used as a microbial pesticide, a seed coating agent, a microbial nutrient, a soil conditioning agent, a compost fertilizing agent, a foliar spray formulation, or a drench-spray formulation.

In the microorganism agent of the present invention, the *Aspergillus niger* F22 strain or the culture broth thereof may be modified into various forms by known methods used in the related art, and may be used in liquid and powdery phases. Preferably, a culture broth or concentrate of the *Aspergillus niger* F22 strain may be adsorbed onto a carrier such as starch, crude proteins, or stone powder, and then dried. The carrier that may be used as a mixture with the culture broth or concentrate of the strain of the present invention may include any carriers used in the related art. Specifically, the carrier that may be used in the present invention includes cereal such as rise, wheat, corn, barley, bean, millet, sorghum, millet, buckwheat, etc., tuber crops such as potato, etc., tuberous roots such as sweet potato, cassava, etc., or processed products thereof (for example, powder), starches derived therefrom, and derivatives thereof. In addition, agar, gelatin, pectate (polygalacturonate), chitosan, carboxymethyl cellulose and derivatives thereof, Geolite, natural wax, natural gum, kaolin, clay minerals such as bentonite, or kieselguhr materials such as Geolite may be used as the carrier. Such various carriers may be used alone, or two or more carriers may be mixed at a proper ratio to obtain a carrier having improved physical properties. When the aforementioned carriers are used, the carriers may be metabolized into nutrients by microorganisms, and may have an increased adhesive property to a surface of a plant because such carriers have very high viscosity.

The present invention may also include a dried product obtained by drying the strain or the strain culture broth, and a biological pesticide including the same. The dried product may be used as a formulation selected from the group consisting of a wettable powder (WP), a granular material (GM), a water-dispersible granule (WG), a granule (GR), a dustable powder (DP), and a water dispersible powder for slurry seed treatment (WS) to prepare a biological pesticide. Such a biotic pesticide formulation has excellent stability and physicochemical properties, compared to conventional liquid formulations, and may be used to control plant diseases.

Among the biotic pesticides, the wettable powder refers to an agricultural pesticide formulation that is in a powdery phase begin to hydrate as soon as they are added to water, and the granular material refers to a formulation in which a culture broth of microorganisms is mixed with or adsorbed onto a solid material, that is, a formulation which does not correspond to the granule and wettable powder formulations. Also, the water-dispersible granule refers to an agricultural pesticide formulation that is in a granular phase and used after being diluted with water, the granule refers to an agricultural pesticide formulation that is in a granular phase and used intact, the dustable powder refers to an agricultural pesticide formulation that is in a powdery phase and used intact, and the water dispersible powders for slurry seed treatment refers to an agricultural pesticide formulation that is in a powdery phase and hydrated for use as a suspension prior to treatment of seeds.

The wettable powder according to one exemplary embodiment of the present invention may include an *Aspergillus niger* F22 strain as an active ingredient, white carbon as a moisture absorbent, sodium bis[2-ethylhexyl]sulfosuccinate as a humectant, sodium lignosulfonate as a dispersing agent, and kaolin as a bulking agent. Preferably the wettable powder may include 10% by weight of the *Aspergillus niger* F22 strain, 1% by weight of white carbon, 1% by weight of sodium bis[2-ethylhexyl]sulfosuccinate, 1% by weight of sodium lignosulfonate, and 87% by weight of kaolin.

The suspension concentrate according to one exemplary embodiment of the present invention is used to control plant-parasitic nematodes. To use the microorganism agent of the present invention to control the plant-parasitic nematodes, the microorganism agent may be uniformly diluted with water, and then sprayed on a crop, a crop seed or a field using a suitable spraying machine such as a motor sprayer. When the suspension concentrate of the present invention is diluted with water, a concentration of the suspension concentrate may be adjusted to be in a range of $10^3$ to $10^5$ cfu/mL, preferably approximately $10^4$ cfu/mL so that the active ingredient can be present at a biologically effective dose, but the present invention is not limited thereto.

Also, the present invention provides a method for controlling plant-parasitic nematodes, which include a step of treating a crop, a crop seed or a field with the nematicidal microorganism agent against plant-parasitic nematodes.

The method for controlling plant-parasitic nematodes may be carried out by dipping a crop or a crop seed in a culture broth obtained by culturing the strain of the present invention or a microorganism agent using the strain, or drenching, that is, spraying the culture broth or the microorganism agent onto the crop or crop seed. In the case of the dipping method, the culture broth and agent may be spread on soil around plants, or the seed may be soaked into the culture broth and agent. Plants that may be applied to the method of the present invention are not particularly limited.

Also, the present invention provides a method for preparation of a nematicidal microorganism agent against plant-parasitic nematodes, which includes culturing the strain. Any methods known in the related art may be used as the method for culturing the *Aspergillus niger* F22 strain and the method for preparation of the microorganism agent but are not particularly to certain methods.

Further, the strain may be cultured in a malt extract broth (MEB) medium, a potato dextrose broth (PDB) medium, a Czapek Dox broth (CDB) medium, or a Sabouraud dextrose broth (SDB) medium, preferably a PDB medium, but the present invention is not limited thereto. In the present invention, after the strain is cultured, an *M. incognita* larva suspension is treated with a culture broth at a concentration of 10% using a 96-well microplate bioassay, and the nematicidal activities are examined after 3 days. As a result, the strain has the highest nematicidal activity because the strain exhibits 94% control activity in the PDB medium. Thereafter, the PDB medium is selected as an optimal medium.

Hereinafter, the present invention will be described in detail with reference to embodiments thereof. However, it should be understood that the following examples are just preferred examples for the purpose of illustration only and is not intended to limit or define the scope of the invention.

Example 1: Selection of strains having nematicidal activity and analysis of nematicidal activities of strains

To select filamentous fungi having an excellent nematicidal activity, the present inventors have received 61 culture filtrates of filamentous fungi to screen their nematicidal activities. Specifically, roots of a tomato plant infected with *Meloidogyne* spp. were washed with running water to remove foreign substances. Thereafter, the well-washed roots were cut into pieces at intervals of 1 cm or less, and put into a blender. Then, a 0.5% sodium hyphochlorite solution was added at such an amount that the roots were immersed, and the roots were ground for one minute. Subsequently, the ground roots were filtered through a 65 μm sieve to remove root debris, and the eggs passed through the sieve were collected through a 25 μm sieve, and then washed with sterilized water several times. A concentration of the collected *Meloidogyne* spp. eggs was measured using a stereoscopic microscope.

The nematicidal activities of the culture filtrates of the 61 fungi were screened using a 96-well microplate bioassay. 45 μL of a *Meloidogyne* spp. larva suspension, which includes approximately 50 larvae, was added to each of holes, and then treated with a 10% culture filtrate. After 3 days of the sample treatment, the nematicidal activity was determined using the following equation. The experiment was performed in triplicate, 10% sterilized water was used as the untreated control, and abamectin was used at a lethal concentration of 1 μg/mL as the control drug.

$$\text{Nematicidal activity (\%)} = [\text{Number of dead nematodes}/(\text{Number of viable nematodes} + \text{Number of deadnematodes})] \times 100$$

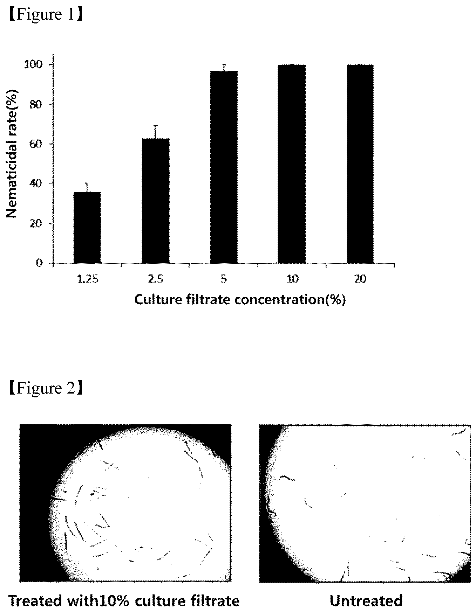

As a result, as shown in FIG. 1, it was revealed that the F22 strain exhibited perfect (100%) nematicidal activity when the nematodes were treated with the 10% culture filtrate, and exhibited potent (96%) nematicidal activity even when the nematodes were treated with the 5% culture filtrate. It can be seen that the nematode larvae did not wriggle in a linearly straight shape when treated with a sample, whereas the nematode larvae were alive because the larvae wriggled along in a flexible curved shape in the untreated group (FIG. 2). Meanwhile, the group in which the nematodes were treated with abamectin used as the control drug exhibited 95% nematicidal activity when treated with 1 μg/mL of abamectin.

Example 2: Identification of strains

The F22 strain having an excellent nematicidal activity was cultured in a PDB medium at 25 °C on shaker at 150 rpm for seven days. To identify the F22 strain, DNA sequences of an internal transcribed spacer (ITS) region and 26S rRNA region were determined and analyzed. Specifically, genomic DNA was extracted using a DNeasy Plant mini kit (Qiagen, Valencia, CA, USA), and used as a PCR template. PCR was performed using a set of primers, ITS1 (5'-TCC GTA GGT GAA CCT GCG C-3': SEQ ID NO: 1) and ITS4 (5'-TCC TCC GCT TAT TGA TAT GC-3': SEQ ID NO: 2), to analyze the DNA sequence of the ITS region of the F22 strain. PCR was performed using a set of primers, NL1 (5'-GCA TAT CAA TAA GCG GAG GAA AAG-3': SEQ ID NO: 3) and NL4 (5'-GGT CCG TGT TTC AAG ACG G-3': SEQ ID NO: 4), to analyze the DNA sequence of the 26S rRNA region.

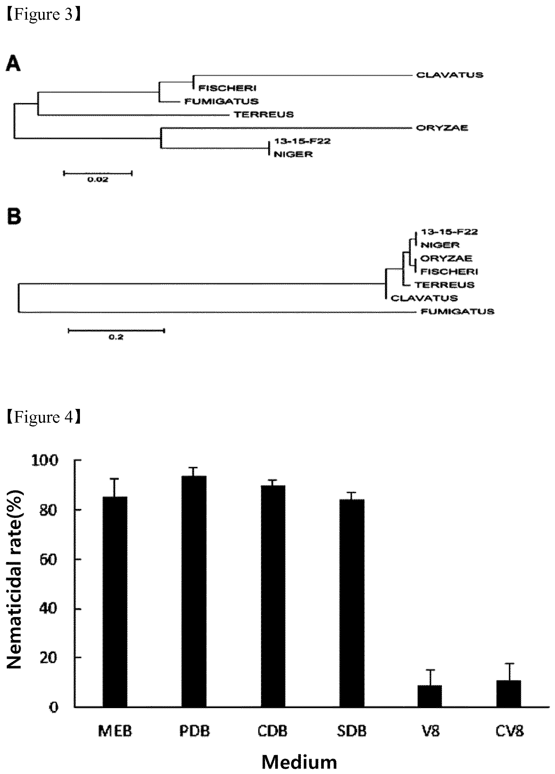

After the ITS region and the 26S rRNA region of the F22 strain were amplified by PCR, the amplified PCR products were purified using a QIAquick PCR purification kit (Qiagen, Hilden, Germany). The analyzed the DNA sequence of the fungal strain was compared with sequences of the registered related strains by searching the database provided by the US Broad Institute. As a result of comparison between the DNA sequences, the strain having the highest homology was identified. As a result, as shown in FIG. 3, it was revealed that both of the two regions were grouped into *Aspergillus niger,* and the F22 strain was identified as *Aspergillus niger* because the ITS region and the 26S rRNA regions had homologies of 99.8% and 98%, respectively.

Example 3: Selection of optimal medium

To select an optimal medium to produce substances having a nematicidal activity from the F22 strain, the nematicidal activities according to the type of the culture medium were examined using 6 media generally used for fungal culture. Five pieces of culture plate (diameter: 6 mm) of the F22 strain were inoculated in six different culture media, and incubated for seven days under conditions of 25 °C and 150 rpm while stirring. In this case, the six media used in this experiment were as follows: A malt extract broth (MEB) medium, a potato dextrose broth (PDB) medium, a Czapek Dox broth (CDB) medium, a Sabouraud dextrose broth (SDB) medium, a V8 juice medium, and a CV8 juice medium (a Clarified V8 juice medium from Campbell Soup Company). The culture broths of the F22 strain cultured in such six media were filtered through sterilized gauze to obtain culture filtrates. Then, a *Meloidogyne* spp. larva suspension was treated with each of the culture filtrates at a concentration of 10% using the aforementioned 96-well microplate bioassay, and the nematicidal activities were examined after 3 days. As a result, the F22 strain had the highest nematicidal activity as the F22 strain exhibited 94% control activity in the PDB medium, as shown in FIG. 4. Thereafter, the PDB medium was selected as an optimal medium.

Example 4: Control activity of culture filtrates against *Meloidogyne incognita*

Disease control activity of the culture filtrate of the *Aspergillus niger* (*A. niger*) F22 strain was investigated by pot culture experiment that includes tomato plants infected with *Meloidogyne incognita.* 400 g of sterilized sand was put into a plastic pot having a diameter of 9.5 cm, and applied to bed soil. Thereafter, a cultivar, 'Seogwang (Monsanto Korea, Inc.), of tomato grown for 3 weeks in the greenhouse was transplanted. *Meloidogyne* spp. eggs were collected from tomato roots infected with *Meloidogyne* spp. according to the aforementioned method, and the 10,000 eggs were inoculated in each of the pots. Then, the F22 strain was cultured in a PDB medium for 7 days under conditions of 25 °C and 150 rpm while stirring, and the culture broth of the F22 strain was filtered through sterilized gauze to obtain a culture filtrate. The culture filtrate was diluted 10-fold and 100-fold with sterilized water, and soil around the tomato roots was then drenched twice with the culture filtrate at an interval of one week. The soil was treated once with a 3,000-fold dilute solution of All Star (a.i., Abamectin 1.8%) as the control, and, in the case of the untreated group, the soil was treated with sterilized water. The experiments were performed in quintuplicate, and roots of the plant were cleanly washed with running water 6 weeks after inoculation of the nematode eggs. Then, a root-knot index was examined according to the method by Taylor and Sasser (1978, Identification and Control of Root-Knot Nematodes (Meloidogyne Species); Department of Plant Pathology, North Carolina State University: Raleigh, NC, 1978; Vol. 2, p 111). The root-knot index was examined at levels 0 to 5 (0: 0%, 1: 1 to 20%, 2: 21 to 40%, 3: 41 to 60%, 4: 61 to 80%, and 5: 81 to 100%).

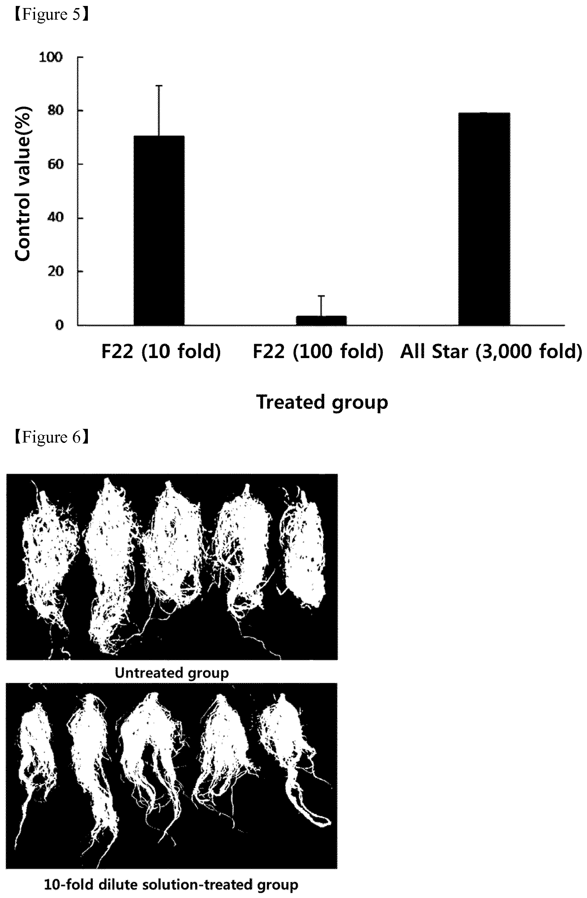

As a result, as shown in FIG. 5, it was revealed that the F22 strain exhibited 71% control activity when *Meloidogyne incognita* was treated with a 10-fold dilute solution of the culture filtrate of the F22 strain, and the vigorous growth of the plant was observed. As described above, it was demonstrated that the *A. niger* F22 strain was considered to be effectively used to control *Meloidogyne incognita*.

Example 5: Examination of control activity of basic formulations using *A. niger* F22 strain against root-knot nematode diseases

1. Preparation of basic formulations including *A. niger* F22 strain

To develop basic formulations using the culture broth of the F22 strain, two suspension formulations (suspension concentrate (SC) and suspension microbial (SM)), two granule formulations (absorbent granule (GR) and powdery granule (GR)), and one wettable powder (WP) formulation were prepared.

① Preparation of *A. niger* F22 suspension concentrate (SC) formulation

A) Preparative prescription: A formulation was prepared using components finally prescribed as listed in the following Table 1.

[Table 1]

| Prescription for preparation of *A. niger* F22 suspension concentrate (SC) formulation | | | |
|---|---|---|---|
| Role | Composition | Input (%) | Form |
| Technical product | *A. niger* F22 SD powder | 10.0 | Brown liquid |
| Surfactant | Wetting/dispersing agent | 3.0 | Colorless liquid |
| Adjuvant | Preservative | 1.5 | Light yellow powder |
| | Antifreeze agent | 5.0 | Colorless liquid |
| | Thickening agent | 0.2 | Yellow powder |
| Bulking agent | Bulking agent | Balance | Colorless powder |
| Total | | 100.0 | - |

B) Process flowchart

1) Wet-ground portion (50%)

A surfactant was sufficiently dispersed in water, and an *A. niger* culture broth was ground, and then mixed with the dispersion. When the bubbles occurred, a small amount of an antifoaming agent was added at divided doses.

2) Thickened portion (50%)

A thickening agent was uniformly dispersed in an antifreeze agent, and a preservative and water were then added thereto, and evenly stirred.

3) Process of mixing product portions

The wet-ground portion and the thickened portion were mixed at a proper ratio (i.e., a recommend ratio of 50:50) to prepare a formulation.

(2) Preparation of *A. niger* F22 suspension microbial (SM) formulation

A) Preparative prescription: A formulation was prepared using components finally prescribed as listed in the following Table 2.

[Table 2]

| Prescription for preparation of *A. niger* F22 suspension microbial (SM) formulation | | | |
|---|---|---|---|
| Role | Composition | Input (%) | Form |
| Technical product | *A. niger* F22 culture broth | 10.0 | Brown liquid |
| Surfactant | Wetting/dispersing agent | 3.0 | Colorless liquid |
| Adjuvant | Preservative | 1.5 | Light yellow powder |
| | Antifreeze agent | 5.0 | Colorless liquid |
| | Thickening agent | 0.2 | Yellow powder |
| Bulking agent | Bulking agent | Balance | Colorless powder |
| Total | | 100.0 | - |

B) Process flowchart

1) Technical product-mixed portion (50%)

A surfactant was sufficiently dispersed in water, and then mixed with a powder obtained by spray-drying the culture broth of *A. niger* F22. When the bubbles occurred, a small amount of an antifoaming agent was added at divided doses.

2) Thickened portion (50%)

A thickening agent was uniformly dispersed in an antifreeze agent, and a preservative and water were then added thereto, and evenly stirred.

3) Process of mixing product portions

The technical product-mixed portion and the thickened portion were mixed at a proper ratio (i.e., a recommend ratio of 50:50) to prepare a formulation.

(3) Preparation of *A. niger* F22 absorbent granule (GR) formulation

A) Preparative prescription: A formulation was prepared using components finally prescribed as listed in the following Table 3.

[Table 3]

| Prescription for preparation of *A. niger* F22 absorbent granule (GR) formulation | | | |
|---|---|---|---|
| Role | Composition | Input (%) | Form |
| Technical product | *A. niger* F22 culture broth | 10.0 | Yellow liquid |
| Bulking agent | Granular kieselguhr | Balance | Light yellow granule |
| Total | | 100.0 | - |

B) Process flowchart

A mixture of *A. niger* F22 culture broth and granular kieselguhr was spraydried to prepare a formulation.

④ Preparation of *A. niger* F22 powdery granule (GR) formulation

A) Preparative prescription: A formulation was prepared using components finally prescribed as listed in the following Table 4.

[Table 4]

| Prescription for preparation of *A. niger* F22 powdery granule (GR) formulation | | | |
|---|---|---|---|
| Role | Composition | Input (%) | Form |
| Technical product | *A. niger* F22 SD powder | 10.0 | yellow liquid |
| Surfactant | Dispersing agent | 2.5 | Light brown liquid |
| | Humectant | 0.5 | light yellow liquid |
| Adjuvant | Disintegrating agent | 1.5 | White powder |
| | Binder | 2.0 | yellow powder |
| Bulking agent | Bulking agent-1 | 25.0 | Gray powder |
| | Bulking agent-2 | Balance | White powder |
| Total | | 100.0 | - |

B) Process flowchart

The technical product, adjuvants, and bulking agents were uniformly mixed, and a proper amount of water was added to a liquid surfactant, and kneaded. Thereafter, the resulting mixture was ground into powder, dried, and screened to prepare a formulation.

⑤ Preparation of *A. niger* F22 wettable powder (WP) formulation

A) Preparative prescription: A formulation was prepared using components finally prescribed as listed in the following Table 5.

[Table 5]

| Prescription for preparation of *A. niger* F22 wettable powder (WP) formulation | | | |
|---|---|---|---|
| Role | Composition | Input (%) | Form |
| Technical product | *A. niger* F22 SD powder | 10.0 | Yellow liquid |
| Surfactant | Dispersing agent-1 | 5.0 | Brown powder |
| | Dispersing agent-2 | 1.0 | White powder |
| | Humectant | 3.0 | White powder |
| Preservative | Preservative | 1.0 | White granule |
| Bulking agent | Bulking agent | Balance | Yellow powder |
| Total | | 100.0 | - |

B) Process flowchart

The technical product, surfactants, an adjuvant, and a bulking agent were mixed and ground to prepare a formulation.

2. Examination of control activity of basic formulations including *A. niger* F22 strain against *Meloidogyne incognita*

*In vivo* control activities of a total of the five formulations thus prepared, that is, suspension concentrate (SC), suspension microbial (SM), absorbent granule (absorbent GR), powdery granule (powdery GR), and wettable powder (WP) formulations, and culture broths thereof against *Meloidogyne incognita* were examined. Specifically, 400 g of sterilized sand was put into a plastic pot (diameter; 9.5 cm), and tomato seedlings grown for 3 weeks in the greenhouse were transplanted according to the method as described above. Ten thousand nematode eggs were inoculated in each of the pots. After an hour, each of the *A. niger* F22 culture broth and the 5 formulations was diluted 100-fold and 50-fold with sterilized water in the case of the formulations excluding the granule formulation, and soil was drenched with each dilute solution at an amount of 20 mL/pot. In this case, the granule formulation was mixed with soil. The sample treatment was performed twice at an interval of one week, and, in the case of the granule formulation, the secondary treatment was performed after the granule formulation was suspended in water. The soil was treated once with a 3,000-fold dilute solution of All Star (a.i., Abamectin 1.8%) as the control, and, in the case of the untreated group, the soil was treated with sterilized water. The experiments were performed in quintuplicate, and roots of the plant were cleanly washed with running water 6 weeks after inoculation of the nematode eggs. Then, a root-knot index was examined according to the method by Taylor and Sasser (1978, Identification and Control of Root-Knot Nematodes (Meloidogyne Species); Department of Plant Pathology, North Carolina State University: Raleigh, NC, 1978; Vol. 2, p 111). The root-knot index was examined at levels 0 to 5 (0: 0%, 1: 1 to 20%, 2: 21 to 40%, 3: 41 to 60%, 4: 61 to 80%, and 5: 81 to 100%).

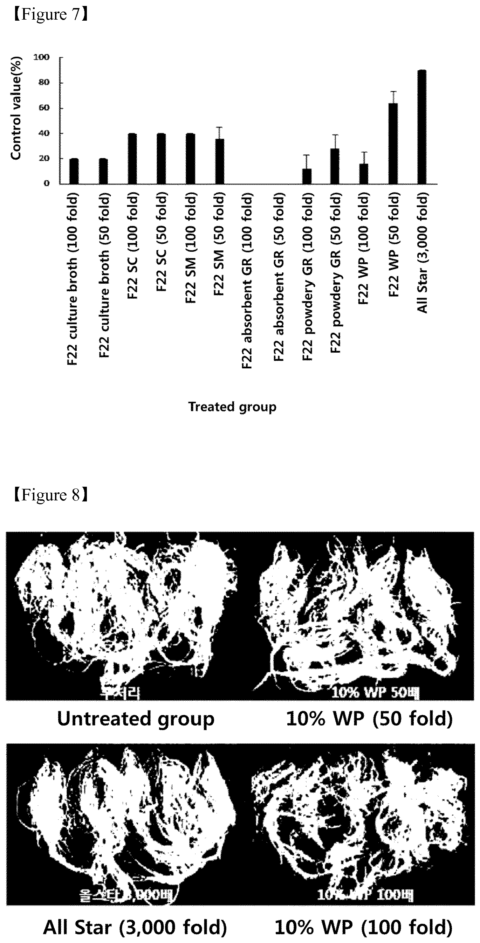

As a result, as shown in FIG. 7, it was revealed that the F22 strain had control values of 16% and 64%, respectively, in the groups in which the soil was treated with the 100-fold and 50-fold dilute solutions of the wettable powder (WP) formulation including the F22 strain, the control values increasing in a concentration-dependent manner. Therefore, it was expected that, when the F22 strain was used to develop products, the granule formulations exhibited superior control activity, compared to when the F22 strain was prepared into the wettable powder formulation.

Example 6: Analysis of control activity of culture filtrates against *Meloidogyne hapla* and *Bursaphelenchus xylophilus*

To check the control activity of the *A. niger* F22 strain against various plant-parasitic nematode diseases, the control activities against *Meloidogyne hapla* and *Bursaphelenchus xylophilus* were examined.

The larvae of *Meloidogyne hapla* and *Bursaphelenchus xylophilus* were kindly provided from the Professor Young Ho KIM's laboratory at the department of Applied Biology of Seoul National University to perform experiments. The nematode larva suspension consisting of J2 stage of *Meloidogyne hapla* or *Bursaphelenchus xylophilus* were prepared at a concentration of 100 larvae/100 $\mu$L. Then, 80 $\mu$L of the nematode larva suspension was put into a 96-well microplate, and 20 $\mu$L of the culture filtrate was added thereto so that the suspension was treated with the culture filtrate to reach a concentration of 20%. Subsequently, 20 $\mu$L of the suspension in the 20% treated group was transferred to wells containing 80 $\mu$L of the nematode larva suspension so that the suspension was treated with the culture filtrate to reach a concentration of 5% at which the suspension was diluted 1/4-fold. In this manner, the nematicidal activities of the culture filtrates against the J2 stage of nematode larvae at concentrations of 20%, 5%, and 1% were examined. After the sample treatment, *Meloidogyne hapla* and *Bursaphelenchus xylophilus* were stored at 25 °C for 24 hours in an incubator, and the nematicidal activity was then determined using the following equation.

$$\text{Nematicidal activity (\%)} = [\text{Number of dead nematodes}/(\text{Number of viable nematodes} + \text{Number of deadnematodes})] \times 100$$

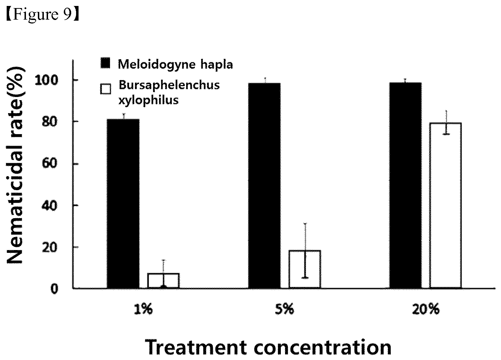

As a result, it was revealed that, when *Meloidogyne hapla* and *Bursaphelenchus xylophilus* were treated with the 20%, 5%, and 1% culture filtrates, respectively, the *A. niger* F22 strain exhibited 99.1%, 98.4%, and 80.9% nematicidal activities against *Meloidogyne hapla,* and exhibited 79.7%, 18.3%, and 7.5% nematicidal activities against *Bursaphelenchus xylophilus.* Therefore, it was confirmed that the *A. niger* F22 strain exhibited excellent nematicidal activity against *M. hapla* and *B. xylophilus* as well as *M. incognita,* particularly exhibited potent nematicidal activity against *Meloidogyne hapla* (FIG. 9).

[Accession Number]

Depository Institution: Korea Research Institute of Bioscience and Biotechnology Accession Number: KCTC12771BP

Filling Date: March 18, 2015

<110> INDUSTRY FOUNDATION OF CHONNAM NATIONAL UNIVERSITY KOREA RESEARCH INSTITUTE OF CHEMICAL TECHNOLOGY Korea Research Institute of Bioscience and Biotechnology

<120> Aspergillus niger F22 strain having nematocidal activity against plant parasitic nematode and uses thereof

<130> PCT1612

<160> 4

<170> KoPatentIn

<210> 1
<211> 19
<212> DNA
<213> Artificial Sequence

<220>
<223> primer

<400> 1
tccgtaggtg aacctgcgc         19

<210> 2
<211> 20
<212> DNA
<213> Artificial Sequence

<220>
<223> primer

<400> 2
tcctccgctt attgatatgc         20

<210> 3
<211> 24
<212> DNA
<213> Artificial Sequence

<220>
<223> primer

<400> 3
gcatatcaat aagcggagga aaag         24

<210> 4
<211> 19
<212> DNA
<213> Artificial Sequence

<220>
<223> primer

<400> 4
ggtccgtgtt tcaagacgg         19

## Claims

1. An *Aspergillus niger* (*A. niger*) F22 strain having a nematicidal activity against the plant-parasitic nematodes *Bursaphelenchus xylophilus* (*B. xylophilus*), and *Meloidogyne* spp., wherein the *A. niger* F22 strain is deposited with accession number KCTC 12771BP.

2. The strain of claim 1, wherein the *Meloidogyne* spp. comprises one or more selected from *Meloidogyne hapla, Meloidogyne javanica, Meloidogyne arenaria,* and *Meloidogyne incognita.*

3. A nematicidal microorganism agent against plant-parasitic nematodes, comprising the strain defined in any one of claims 1 to 2, or a spore, a fungal hyphal mass or a culture broth thereof as an active ingredient.

4. The nematicidal microorganism agent of claim 3, wherein the microorganism agent is prepared into a formulation selected from the group consisting of suspension concentrate (SC), suspension microbial (SM), absorbent granule (absorbent GR), powdery granule (powdery GR), dustable powder (DP), water-dispersible granule (WG), water-dispersible powder for slurry seed treatment (WS), and a wettable powder (WP).

5. The nematicidal microorganism agent of claim 4, wherein the microorganism agent is prepared into a wettable powder formulation.

6. A method for controlling plant-parasitic nematodes, comprising:
a step of treating a crop, a crop seed, or a field with the nematicidal microorganism agent against plant-parasitic nematodes defined in any one of claims 3 to 5.

7. A method for preparation of a nematicidal microorganism agent against plant-parasitic nematodes, comprising:
a step of culturing the strain defined in any one of claims 1 to 2.

8. The method of claim 7, wherein the strain is cultured in a potato dextrose broth (PDB) medium.

**Patentansprüche**

1. *Aspergillus niger* (*A. niger*) F22 Stamm, der eine nematizide Aktivität gegen die Pflanzen-Parasiten-Nematoden *Bursaphelenchus xylophilus* (*B. xylophilus*), und *Meloidogyne* spp. aufweist, wobei der *A. niger* F22 Stamm mit der Hinterlegungsnummer KCTC 12771BP abgelegt ist.

2. Stamm nach Anspruch 1, wobei *Meloidogyne* spp. eines oder mehr umfasst, ausgewählt aus *Meloidogyne hapla, Meloidogyne javanica, Meloidogyne arenaria,* und *Meloidogyne incognita.*

3. Nematizides Mikroorganismenmittel gegen Pflanzen-Parasiten-Nematoden, umfassend einen Stamm nach einem der Ansprüche 1 bis 2, oder eine Spore, eine pilzartige hyphale Masse oder ein Kulturbrei davon als Wirkstoff.

4. Nematizides Mikroorganismenmittel nach Anspruch 3, wobei das Mikroorganismenmittel in einer Formulierung aufbereitet wird, die aus der Gruppe ausgewählt wird, die aus einem Suspensionskonzentrat (SC), mikrobieller Suspension (SM), einem absorbierenden Granulat (absorbierendes GR), einem pulverigen Granulat (pulveriges GR), einem verstäubbaren Pulver (DP), einem wasserdispergierbaren Granulat (WG), einem wasserdispergierbaren Pulver zur Schlammimpfbehandlung (WS), und einem benetzbaren Pulver (WP) besteht.

5. Nematizides Mikroorganismenmittel nach Anspruch 4, wobei das Mikroorganismenmittel in einer benetzbaren Pulverformulierung aufbereitet wird.

6. Verfahren zum Kontrollieren von Pflanzen-Parasiten-Nematoden, umfassend:
einen Schritt des Behandelns einer Feldsaat mit dem nematiziden Mikroorganismenmittel gegen Pflanzen-Parasiten-Nematoden nach einem der Ansprüche 3 bis 5.

7. Verfahren zum Aufbereiten eines nematiziden Mikroorganismenmittels gegen Pflanzen-Parasiten-Nematoden, umfassend:
einen Schritt des Kultivierens des Stammes nach einem der Ansprüche 1 bis 2.

8. Verfahren nach Anspruch 7, wobei der Stamm in einem Kartoffel-Dextrose-Brühemedium (PDB) kultiviert wird.

**Revendications**

1. Souche d'*Aspergillus niger* (*A. niger*) F22 ayant une activité nématicide contre les nématodes phyto-parasitaires *Bursaphelenchus xylophilus* (*B. xylophilus*), et *Meloidogyne* spp., dans laquelle la souche d'*A. niger* F22 est déposée sous le numéro d'accès KCTC 12771BP.

2. Souche selon la revendication 1, dans laquelle *Meloidogyne* spp. comprend un ou plusieurs sélectionnés parmi *Meloidogyne hapla, Meloidogyne javanica, Meloidogyne arenaria,* et *Meloidogyne incognita.*

**3.** Agent de microorganisme nématicide contre des nématodes phyto-parasitaires, comprenant la souche définie dans l'une quelconque des revendications 1 à 2, ou une spore, une masse de l'hyphe fongique ou un bouillon de culture de celles-ci sous la forme d'un ingrédient actif.

**4.** Agent de microorganisme nématicide selon la revendication 3, dans laquelle l'agent de micro-organisme est préparé en une formulation sélectionnée dans le groupe consistant en suspension concentrée (SC), suspension microbienne (SM), granulé absorbant (GR absorbant), granulé pulvérulent (GR pulvérulent), poudre pulvérisable (DP), granulé dispersible dans l'eau (WG), poudre dispersible dans l'eau pour bouillie de traitement des semences (WS), et poudre mouillable (WP).

**5.** Agent de microorganisme nématicide selon la revendication 4, dans laquelle l'agent de micro-organisme est préparé en une formulation de poudre mouillable.

**6.** Procédé de contrôle de nématodes phyto-parasitaires, comprenant :
une étape de traitement d'une culture, d'une semence de culture, ou d'un champ avec l'agent de micro-organisme nématicide contre des nématodes phyto-parasitaires défini dans l'une quelconque des revendications 3 à 5.

**7.** Procédé de préparation d'un agent de micro-organisme nématicide contre des nématodes phyto-parasitaires, comprenant :
une étape de culture de la souche définie dans l'une quelconque des revendications 1 à 2.

**8.** Procédé selon la revendication 7, dans lequel la souche est cultivée dans un milieu de bouillon de dextrose à la pomme de terre (PDB).

【Figure 1】

【Figure 2】

Treated with10% culture filtrate          Untreated

【Figure 3】

【Figure 4】

【Figure 5】

【Figure 6】

Untreated group

10-fold dilute solution-treated group

【Figure 7】

【Figure 8】

Untreated group      10% WP (50 fold)

All Star (3,000 fold)      10% WP (100 fold)

【Figure 9】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20060002789
- KR 20100116562

- CN 101182469
- JP 2005082530 A

### Non-patent literature cited in the description

- **CABONI et al.** *J. Agric. Food Chem.,* 2013, vol. 61, 1794-1803
- **ZHAO et al.** Antagonistic action of Bacillus subtillis strain SG6 on Fusarium graminearum. *PLoS one,* 2014, vol. 9 (3), e92486

- **TAYLOR ; SASSER.** *Identification and Control of Root-Knot Nematodes (Meloidogyne Species),* 1978
- Department of Plant Pathology. North Carolina State University, 1978, vol. 2, 111